(19) Europäisches Patentamt
European Patent Office
Office européen des brevets

(11) Numéro de publication : **0 511 038 A1**

(12) # DEMANDE DE BREVET EUROPEEN

(21) Numéro de dépôt : **92400878.2**

(51) Int. Cl.$^5$ : **C07C 53/12,** C07C 51/56

(22) Date de dépôt : **30.03.92**

La demande, qui etait incomplète au moment du dêpot, est publiée telle quelle (article 93 (2) CBE). Le passage de la description ou des revendications qui comporte manifestement une omission est présenté comme tel.

(30) Priorité : **25.04.91 FR 9105145**

(43) Date de publication de la demande :
**28.10.92 Bulletin 92/44**

(84) Etats contractants désignés :
**AT BE CH DE DK ES FR GB GR IT LI LU NL PT SE**

(71) Demandeur : **SOLLAC**
**Immeuble Elysées La Défense, 29 Le Parvis**
**F-92800 PUTEAUX (FR)**

(72) Inventeur : **Castanet, Yves**
**11, Allée Pierre Bonnard**
**F-59510 Hem (FR)**
Inventeur : **Seuillet, Bruno**
**Résidence Thallès, 94, rue de Paris**
**F-60000 Compiègne (FR)**
Inventeur : **Mortreux, André**
**17, rue Léon Gambetta**
**F-59510 Hem (FR)**
Inventeur : **Petit, Francis**
**13, Allée de la Clairière**
**F-59650 Villeneuve d'Asq (FR)**

(74) Mandataire : **Varady, Peter et al**
**Cabinet Lavoix 2, Place d'Estienne d'Orves**
**F-75441 Paris Cedex 09 (FR)**

(54) **Procédé de préparation de l'anhydride acétique à partir du formiate de méthyle.**

(57) L'invention a pour objet un procédé de préparation de l'anhydride acétique, caractérisé en ce que l'on fait réagir sur le formiate de méthyle un gaz comprenant principalement du CO, sous une pression d'au moins 1MPa et en présence de
a) un catalyseur à base de rhodium,
b) deux promoteurs iodés de nature différente, successivement ionique et covalent, et
c) un amide cyclique N-substitué en tant que solvant.

EP 0 511 038 A1

$$CH_3OH + CO \xrightarrow{Rh} CH_3COOH$$

$$CH_3COOH + CH_3OH \longrightarrow CH_3CO_2CH_3 + H_2O$$

$$CH_3CO_2CH_3 \xrightarrow[CO]{Rh} CH_3COOCOCH_3$$

- via le formiate de méthyle

$$CH_3OH + CO \xrightarrow{NaOCH_3} HCO_2CH_3$$

$$HCO_2CH_3 \longrightarrow CH_3COOCOCH_3 \; ?$$

Nous avons maintenant trouvé que cette dernière réaction est réalisable, de sorte que l'anhydride acétique est accessible à partir de méthanol, produit industriel peu onéreux.

La présente invention a ainsi pour objet un procédé de préparation de l'anhydride acétique, caractérisé en ce que l'on fait réagir sur le formiate de méthyle un gaz comprenant principalement du CO, sous une pression d'au moins 1MPa et en présence de

a) un catalyseur à base de rhodium,

b) deux promoteurs iodés de nature différente, successivement ionique et covalent, et

c) un amide cyclique N-substitué en tant que solvant.

On opère avantageusement à une température de 100 à 260°C (température au delà de laquelle la stabilité du catalyseur devient problèmatique) et préférentiellement de 150 à 210°C.

Comme gaz comprenant principalement du CO, on entend le CO pur ou un gaz riche en CO tel que le gaz d'acierie, ce qui permet de valoriser ce gaz. Un gaz d'acierie a par exemple les compositions suivantes : CO=74,5%,$CO_2$=14,9%,$H_2$=2%,$N_2$=8,3%,$O_2$=O,2%.

La pression doit être d'au moins 1MPa. Cette pression peut être maintenue constante ou avantageusement augmentée au cours de la réaction sans qu'il soit nécessaire de dépasser 15 MPa.

Comme exemple de catalyseurs à base de rhodium on peut citer $RhCl_3,3H_2O$, $Rh_2Cl_2(CO)_4$, $RhCOCl(PPh_3)_2$, $RhI_3$ ... cette liste n'étant pas limitative, tous les sels ou complexes du Rh essayés s'étant révélés actifs.

Comme indiqué précédemment, on opère dans un solvant particulier constitué d'un amide cyclique N-substitué. En effet, la nature du solvant est déterminante car, sans solvant ou dans des solvants usuels autres que des amides cycliques N-substitués, la sélectivité en anhydride acétique est beaucoup plus faible, l'acide acétique devenant généralement le produit principal de la réaction par isomérisation du formiate de méthyle. Comme exemple d'amide N-substitué on peut citer le diméthyl-imidazole, la N-éthylpyrrolidone ou préférentiellement la N-méthylpyrrolidone.

La proportion de solvant par rapport au formiate de méthyle de départ ne devra pas être inférieure à 2 volumes de solvant pour 3 volumes de formiate sous peine de voir augmenter la production d'acide acétique. Au delà de cette valeur la quantité de solvant peut être choisie dans une large gamme, étant entendu toutefois qu'à performance équivalente, on tendra à utiliser la quantité la plus faible possible de solvant de manière à limiter le volume réactionnel et à simplifier les problèmes de séparation des produits formés à l'issue de la réaction avec le solvant. On utilisera de préférence des proportions de solvant par rapport au formiate allant de 1/1 à 3/1 (v/v).

L'emploi de promoteurs iodés est aussi indispensable. Deux types de promoteurs iodés doivent obligatoirement être utilisés successivement dans le courant d'une même réaction.

En début de réaction, le milieu doit renfermer un iodure exclusivement ionique, ce iodure ionique pouvant être minéral, notamment alcalin (par exemple LiI,NaI...) ou alcalino terreux. Ce promoteur iodé ionique peut également être obtenu in situ par le mélange d'un composé organique covalent de l'iode (un iodure d'alkyle par exemple) et d'une phosphine ou d'une amine, conduisant à l'iodure de phosphonium ou d'ammonium quaternaire correspondant.

La teneur initiale en iodure ionique doit être ajustée en fonction de la pression initiale de CO; plus la pression de CO est importante plus la concentration en iodure ionique doit être élevée; elle sera notamment dans une plage allant de 0,1 à 0,5 mole/l.

D'autre part, en cours de réaction un composé covalent de l'iode doit être rajouté au milieu réactionnel. Ceci pourra être réalisé sans qu'il soit pour autant nécessaire d'arrêter la réaction, par exemple à l'aide d'une pompe doseuse haute pression ou grâce à un système de ballast. Comme exemple de composé covalent de l'iode, on peut citer les iodures organiques (iodure d'alkyle) et préférentiellement
$CH_3I$, l'iode moléculaire $I_2$ ou l'acide iodhydrique.

La teneur en iodure covalent sera notamment de 0,15 à 1 mole/l.

Enfin, il est souhaitable d'utiliser un promoteur métallique tel que $Cr(CO)_6$ , bien que non indispensable à la production d'anhydride acétique, cet ajout conduit à une augmentation de l'activité. Ce promoteur est avantageusement utilisé à une concentration allant de $5.10^{-3}$ à $3.10^{-2}$ mole/l.

La réaction selon l'invention est effectuée de préférence sans présence substantielle d'eau, car la présence d'eau a pour effet de diminuer la sélectivité en anhydride acétique.

Les exemples suivants illustrent le procédé selon l'invention .

<u>EXEMPLE 1</u> :

Dans un autoclave de 100 $cm^3$ en acier inoxydable, sont introduits :
- 0,03 g ($1,2.10^{-4}$ mole) de $RhCl_3$, $3H_2O$
- 1 g ($7,46.10^{-3}$ mole) de LiI
- 10 mg ($0,45.10^{-3}$ mole) de $Cr(CO)_6$
- 15 g de formiate de méthyle en solution dans 15 $cm^3$ de N-méthylpyrrolidone (NMP).

L'autoclave est pressurisé à 10 bar (1MPa) de CO, puis chauffé à 190°C et mis sous agitation lorsque la température s'est stabilisée. Au bout de 6 h de réaction, 1,14 g ($8.10^{-3}$ mole) de $CH_3I$ dissous dans 5 $cm^3$ de NMP sont rapidement injectés au milieu réactionnel avec une pompe doseuse haute pression, et la pression de CO est augmentée de 30 bar (3 MPa), ce qui porte la pression totale à chaud à 80 bars (8 MPa). Après 4 h supplémentaires de réaction, le chauffage et l'agitation sont arrêtés et le mélange réactionnel est analysé après refroidissement par chromatographie en phase gazeuse.

Les résultats sont les suivants (exprimés en % molaire des différents constituants dans le mélange) :

$$HCOOCH_3 = 1,5$$
$$CH_3COOCH_3 = 14$$
$$CH_3COOH = 19,5$$
$$CH_3COOCOCH_3 = 48$$

<u>EXEMPLE 2</u> :

On reproduit l'exemple 1, si ce n'est que l'autoclave est pressurisé initialement à 40 bar (4MPa) de CO au lieu de 10 bar, et il n'est plus introduit de CO en cours de réaction.

Les résultats suivants sont obtenus après 10h de réaction :

$$HCOOCH_3 = 3$$
$$CH_3COOCH_3 = 12$$
$$CH_3COOH = 35$$
$$CH_3COOCOCH_3 = 41$$

<u>EXEMPLES 3 et 4</u> : Influence de la quantité de $CH_3I$ introduit.

On reproduit l'exemple 1 en faisant varier la quantité de $CH_3I$ injectée en cours de réaction.

| Ex. | Quantité de $CH_3I$(mmoles) | Produits % $HCOOCH_3$ | $CH_3COOCH_3$ | $CH_3COOH$ | $CH_3COOCOCH_3$ |
|---|---|---|---|---|---|
| 1 | 8 | 1,5 | 14 | 29,5 | 48 |
| 3 | 4 | 2 | 47 | 30 | 12 |
| 4 | 16 | 0 | 11,5 | 24 | 54 |

EXEMPLES 5 et 6 : Influence de la pression de CO

On reproduit l'exemple 1 en modifiant la quantité de CO introduit de façon à obtenir la pression de CO à chaud figurant dans le tableau suivant.

| Ex. | p(CO) (à chaud) | Produits % $HCOOCH_3$ | $CH_3COOCH_3$ | $CH_3COOH$ | $CH_3COOCOCH_3$ |
|---|---|---|---|---|---|
| 1 | 8 MPa | 1,5 | 14 | 29,5 | 48 |
| 5 | 11 " | 1 | 11 | 28 | 50 |
| 6 | 5 " | 2 | 46 | 30 | 2 |

EXEMPLES 6 et 7 : Influence du moment d'introduction de l'iodure covalent :

| Ex. | Temps d'introduction de $CH_3I$ | Produits % $HCOOCH_3$ | $CH_3COOCH_3$ | $CH_3COOH$ | $CH_3COOCOCH_3$ |
|---|---|---|---|---|---|
| 1 | 6 h | 1,5 | 14 | 29,5 | 48 |
| 6 | 3 h | 2,5 | 10 | 83,5 | 4 |
| 7 | 8 h | 1 | 51 | 34 | 14 |

## Revendications

1. Procédé de préparation de l'anhydride acétique, caractérisé en ce que l'on fait réagir sur le formiate de méthyle un gaz comprenant principalement du CO, sous une pression d'au moins 1MPa et en présence

de

a) un catalyseur à base de rhodium,
b) deux promoteurs iodés de nature différente, successivement ionique et covalent, et
c) un amide cyclique N-substitué en tant que solvant.

**2.** Procédé selon la revendication 1, caractérisé en ce que l'on opère à une température de 100 à 260°C.

**3.** Procédé selon la revendication 2, caractérisé en ce que la température est de 150 à 210°C.

**4.** Procédé selon l'une des revendications 1 à 3, caractérisé en ce que le solvant est la N-méthylpyrrolidone.

**5.** Procédé selon l'une des revendications 1 à 4, caractérisé en ce que la proportion de solvant par rapport au formiate de méthyle est supérieure à 2/3 (v/v).

**6.** Procédé selon la revendication 5, caractérisé en ce que la proportion de solvant par rapport au formiate est de 1/1 à 3/1 (v/v).

**7.** Procédé selon l'une des revendications 1 à 6, caractérisé en ce que le premier promoteur iodé est un iodure alcalin ou alcalino-terreux.

**8.** Procédé selon l'une des revendications 1 à 6, caractérisé en ce que le premier promoteur iodé est un mélange d'un composé organique covalent de l'iode avec une phosphine ou une amine.

**9.** Procédé selon la revendication 7 ou 8, caractérisé en ce que le promoteur iodé est utilisé à une concentration de 0, 1 à 0,5 mole/l.

**10.** Procédé selon l'une des revendications 1 à 6, caractérisé en ce que le second promoteur iodé est un composé organique covalent de l'iode.

**11.** Procédé selon la revendication 10, caractérisé en ce que le promoteur iodé est utilisé à une concentration de 0,15 à 1 mole/l.

**12.** Procédé selon l'une des revendications 1 à 11, caractérisé en ce que l'on utilise un promoteur métallique à une concentration de $5.10^{-3}$ à $3.10^{-2}$ mole/l.

**13.** Procédé selon la revendication 12, caractérisé en ce que le promoteur métallique est $Cr(CO)_6$

Office européen des brevets

# RAPPORT DE RECHERCHE EUROPEENNE

Numero de la demande

EP 92 40 0878

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | US-A-3 839 428 (NOBUO ISOGAI)<br>* colonne 3, ligne 23 - ligne 43 *<br>* colonne 4, ligne 33 - ligne 47 *<br>* colonne 5, ligne 24 - ligne 33; revendications 1-7 *<br>----- | 1-4,7 | C07C53/12<br>C07C51/56 |

| DOMAINES TECHNIQUES RECHERCHES (Int. Cl.5 ) |
|---|
| C07C |

Le présent rapport a été établi pour toutes les revendications

| Lieu de la recherche | Date d'achèvement de la recherche | Examinateur |
|---|---|---|
| LA HAYE | 06 JUILLET 1992 | KLAG M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)